Europäisches Patentamt

European Patent Office

Office européen des brevets

(11) Publication number: **0 216 138 B1**

# EUROPEAN PATENT SPECIFICATION

(45) Date of publication of patent specification: **18.12.91**  (51) Int. Cl.⁵: **A61B 1/00**, G02B 7/02, G02B 6/38

(21) Application number: **86111455.1**

(22) Date of filing: **19.08.86**

(54) Endoscope.

(30) Priority: **26.08.85 JP 130693/85**

(43) Date of publication of application:
**01.04.87 Bulletin 87/14**

(45) Publication of the grant of the patent:
**18.12.91 Bulletin 91/51**

(84) Designated Contracting States:
**AT BE CH DE FR GB IT LI NL SE**

(56) References cited:
**EP-A- 0 036 814**
**DE-A- 3 341 876**
**DE-A- 3 425 534**
**DE-A- 3 516 164**

(73) Proprietor: **OLYMPUS OPTICAL CO., LTD.**
**43-2, 2-chome, Hatagaya Shibuya-ku**
**Tokyo 151(JP)**

(72) Inventor: **Yamamoto, Tsutomu**
**B-705 Takao Park Haitsu 1231-19, Hatsuzawa-machi**
**Hachioji-shi Tokyo(JP)**

(74) Representative: **Kennedy, Victor Kenneth et al**
**G.F. REDFERN & CO. Marlborough Lodge 14**
**Farncombe Road**
**Worthing West Sussex BN11 2BT(GB)**

## Description

This invention relates to endoscopes and more particularly to an endoscope removably fitted at a tip portion of the endoscope inserting portion with an attachment for changing a viewing angle and visual field direction, and protecting the tip portion.

There is recently extensively used a (medical) endoscope whereby an elongated inserting portion can be inserted into a body cavity to observe the interior of the body cavity or any treating tool can be used for such treatment as therapy. In addition to such endoscopes, there exists also industrial endoscopes which can be inserted into an instrument or piping to inspect or treat its interior. Among such endoscopes, there are flexible endoscopes wherein the inserting portion is flexible, and rigid endoscopes wherein the inserting portion is rigid.

Now, when an endoscope is inserted, for example, into a body cavity, an illuminating light is radiated toward an object position within the body cavity, reflected light from the object is formed into an image by an objective lens and the image is transmitted to an eyepiece through image guide fibres or the like so that the object can be sighted and observed. Therefore, depending on the object to be observed, it may be necessary to change a viewing angle or visual field direction and to protect the tip portion of the inserting portion or the object.

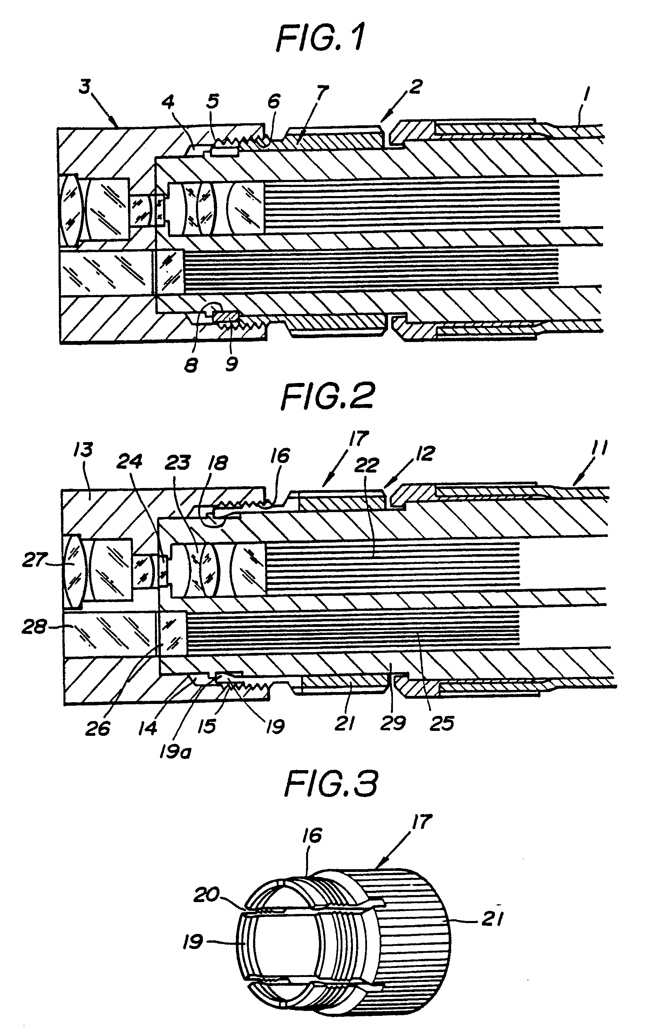

Therefore, there is already known a device whereby a tip portion of an endoscope inserting portion can be removably fitted with an attachment for changing a viewing angle and visual field direction or protecting the tip portion. An example of it is shown in Figure 1.

In Figure 1, the reference numeral 1 represents an inserting portion of an endoscope and 2 represents a tip portion of the endoscope inserting portion. An attachment 3 for changing a viewing angle and visual field direction and protecting the tip portion 2 can be removably fitted to this tip portion 2. This attachment 3 has a recess 4 in which the endoscope tip portion 2 is to be engaged in the rear part, that is, on the endoscope connecting side and has a female thread 5 provided on the inner peripheral wall of the recess 4. On the other hand, a tubular connecting member 7 having a male thread 6 provided to be screwed with the above mentioned female thread 5 is rotatably fitted on the outer periphery of the endoscope tip portion. The male thread 6 of the connecting member 7 and the female thread 5 of the attachment 3 are engaged with each other and the connecting member 7 is rotated to pull in and fix the attachment 3 with the screws.

Now, in this formation, as a means whereby the tubular connecting member 7, which is removably fitted to the above mentioned endoscope tip portion 2 to fix the attachment 3, is held by the tip portion 2 so as not to be pulled off and dropped, conventionally a peripheral groove 8 has been formed in the tip portion 2 adjacent the end of the connecting member 7 and a C-ring 9 has been fitted in this groove 8.

However, in the connecting member holding means using the above mentioned conventional C-ring, in order that the C-ring may not be removed by an external force when the attachment is not fitted, it is necessary to elevate the dimensional precision of the C-ring. That is to say, the C-ring is in a form so plastically deformable that, when it is deformed, the extent to which it engages on the groove will be reduced and the C-ring will be likely to be removed. Therefore, it is necessary to take special care in working and storing the parts of the C-rings and assembling the products.

Tip assemblies for endoscope devices are seen to be suggested in the gazettes of Japanese utility model publications numbers 59/31210 and 57/87704. In these prior art examples, a connecting member is rotatably and nonremovably fitted to the tip portion of an endoscope and a thread provided on the connecting member and a thread of the attachment are screwed with each other to screw and fix this attachment onto the end portion of the endoscope. In these prior art examples, the outer peripheral diameter of the endoscope tip portion in front of this connecting member is made larger or a flange is formed as a means whereby the connecting means for fixing the attachment to the endoscope tip portion is held in the endoscope tip portion so as not to be removable.

An object of this invention is to provide an endoscope wherein a tubular member for removably fixing an attachment to a tip portion of an endoscope can be positively and rotatably locked on the endoscope tip portion and the parts can be easily made, stored, assembled and reduced.

Another object of this invention is to provide an endoscope device wherein the above mentioned connecting member can be easily fitted to the endoscope tip portion from the tip end and therefore the connecting member can be easily assembled to the endoscope.

Briefly, the endoscope according to the present invention includes an attachment having in its rear part a recess in which an inserting portion of an endoscope is engaged, a female thread being provided on the inner peripheral wall of the recess and removably connected and fixed to a connecting member mounted to the tip portion, the connecting member being rotatably mounted on the tip portion of the inserting portion of the endoscope and having a male thread engaged with the female thread of the attachment on the outer periphery on the tip side to pull in and fix the attachment, a circumferential groove being formed on the outer periphery of the tip portion of the endoscope inserting portion adjacent the leading end of the connecting member, and a number of engaging members extending from the leading end portion of the connecting member so as to be engaged in the above mentioned groove of the endoscope tip portion and a number of axial slots being formed in the leading end portion of the connecting member to provide an elastic deformability thereto.

This and other objects and advantages of the present invention will become further apparent from the following detailed explanation in which reference to the Figures will be made. In the Figures,

Figure 1 is a sectional view showing a known endoscope tip assembly,

Figure 2 is a sectioned view showing a tip portion of an endoscope according to the present invention, and

Figure 3 is a perspective view showing the connecting member of Figure 2.

In Figures 2 and 3, the reference numeral 11 represents an inserting portion of an endoscope and 12 represents its tip portion. An attachment 13 for changing a viewing angle and visual field direction or protecting an objective lens and object so as not to be hurt can be removably connected and fixed to this tip portion 12. This attachment 13 has a recess 14 in which an endoscope tip forming portion 29 is to be engaged in the rear part, that is, on the endoscope connecting side and has a female thread 15 provided on the inner peripheral wall of the recess 14. On the other hand, a tubular connecting member 17 having a male thread 16 to be engaged with the female thread 15 of the above mentioned attachment 13 provided on the tip side is rotatably fitted on the outer periphery of the endoscope tip forming portion 29. Also, a groove 18 is peripherally formed on the outer periphery of the endoscope tip forming portion 29 adjacent the leading end of this fitted connecting member 17.

The above mentioned connecting member 17 has a number of engaging members 19 having a projecting portion 19a projecting radially inwardly so as to be engaged with the groove 18 in the position of the groove 18 when fitted to the endoscope tip portion, and has a plurality of thin axial slots 20 to give the engaging member 19 an elastic deformability in a radial direction. The rear portion of this connecting member 17 is an operating portion 21 having knurls.

In Figure 2, the reference numeral 22 represents image guiding fibres, 23 represents and objective lens, 24 represents a cover glass, 25 represents light guiding fibres, 26 represents a cover glass, 27 represents an objective system of the attachment and 28 represents an illuminating system.

In the formation of the assembly, when the tubular connecting member 17 is fitted to the endoscope tip forming portion 29 from the tip side and the engaging member 19 of the connecting member 17 passes over the edge part of the groove 18, it will be elastically deformed in a diameter expanding direction but, on the other hand, when the engaging members 19 pass over the edge part of the groove 18 and come to the groove 18, the engaging members 19 will return to the original diameter and will be engaged with the groove 18 and the connecting member 17 will be able to be rotatably and positively locked in the endoscope tip forming portion 29. Therefore, once the above mentioned connecting member 17 is fitted to the endoscope tip forming portion 29, even if an ordinarily expected external force is applied, the leading end of the connecting member 17 having the engaging members 19 will be very unlikely to be expanded and, even if one side of the leading end divided by the slots 20 is expanded, unless the opposite side is simultaneously expanded, the engaging members 19 will not be disengaged from the groove 18. Also, this connecting member is more rigid in structure than the C-ring and requires no working dimensional precision and the stock of the parts is easy to control. Further, during assembly the connecting member 17 only requires to be pushed onto the outer periphery of the endoscope tip forming portion, for the endoscope tip and connecting member 17 to be assembled, and therefore the manufacture of the device is simple.

## Claims

1. An endoscope including an attachment for changing the viewing angle or the visual field direction and for protecting a tip portion of the endoscope insertion portion, said attachment being removably fitted at a tip portion of the endoscope inserting portion, the attachment (13) having in the rear part a recess (14) in which the tip portion (29) of an endoscope inserting portion (11) is engaged, and having a female thread (15) provided on the inner peripheral wall of this recess (14) and a tubular connecting member (17) rotatably and nonremovably fitted to the outer periphery of the tip portion (29) of the endoscope inserting portion (11), having on its outer periphery a male thread (16) engageable with the female thread (15) of the attachment (13) for pulling in and fixing the attachment, and the endoscope having a groove (18) which extends circumferentially about the outer periphery of the tip portion (29) of the endoscope inserting portion (11) and characterised by a plurality of radially inwardly projecting engaging members (19a) provided to extend from the leading end (19) of the connecting member (17), the connecting member having a plurality of slots (20) formed in the axial direction in the leading end (19) of the connecting member (17) to impart to the connecting member an elastic deformability in a radial direction and the radially inwardly projecting engaging members (19a) being permanently engaged in the peripheral groove (18) to prevent axial movement of the connecting member (17) relative thereto.

2. An endoscope device according to Claim 1, wherein said connecting member (17) has finger gripping means (21) formed on its outer surface behind the male threaded region (16).

## Revendications

1. Endoscope comprenant un accessoire afin de modifier l'angle de visée ou la direction du champ visuel et pour protéger une extrémité de la partie d'introduction de l'endoscope, ledit accessoire étant ajusté de façon amovible à une extrémité de la partie d'introduction de l'endoscope, l'accessoire (13) possédant à la partie postérieure un évidement (14) dans lequel est engagée l'extrémité (29) d'une partie d'introduction de l'endoscope (11), et possédant un taraudage (15) prévu sur la paroi périphérique intérieur de cet évidement (14) et un élément de liaison tubulaire (17) ajusté de façon rotative et inamovible sur la périphérie extérieure de l'extrémité (29) de la partie d'introduction de l'endoscope (11), possédant sur sa périphérie extérieure un filetage (16) pouvant coopérer avec le taraudage (15) de l'accessoire (13) pour introduire et fixer l'accessoire, et l'endoscope possédant une gorge (18) s'étendant circonférentiellement autour de la périphérie extérieure de l'extrémité (29) de la partie d'introduction de l'endoscope (11) et caractérisé par une pluralité d'éléments de contact (19a) saillant radialement vers l'intérieur prévus pour s'étendre depuis l'extrémité antérieure (19) de l'élément de liaison (17), l'élément de liaison possédant une pluralité de fentes (20) pratiquées dans la direction axiale dans l'extrémité antérieure (19) de l'élément de liaison (17) afin de conférer à l'élément de liaison une déformabilité élastique dans une direction radiale et les éléments de contact (19a) saillants radialement vers l'intérieur étant engagés en permanence dans la gorge périphérique (18) afin d'empêcher un déplacement axial de l'élément de liaison (17) par rapport à celle-ci.

2. Endoscope selon la revendication 1, dans lequel ledit élément de liaison (17) possède des moyens de préhension en forme de doigts (21) réalisés sur sa surface extérieure derrière la région filetée (16).

## Patentansprüche

1. Ein Endoskop einschließlich eines Zusatzes zur Veränderung des Sichtwinkels oder der Gesichtsfeldrichtung sowie zum Schutz eines Kopfteils des Endoskopeinführteils, wobei der genannte Zusatz (13) lösbar mit einem Kopfteil des Endoskopeinführteils verbunden ist und im hinteren Teil eine Ausnehmung (14) aufweist, in welche der Kopfteil (29) eines Endoskopeinführteils (11) hineinragt, und ein an der inneren Umfangswand dieser Ausnehmung (14) angebrachtes Innengewinde (15) aufweist, sowie einschließlich eines rohrförmigen Verbindungsteils (17), das drehbar und unlösbar am äußeren Umfang des Kopfteils (29) des Endoskopeinführteils (11) angebracht ist und auf seinem äußeren Umfang ein Außengewinde (16) trägt, welches in das Innengewinde (15) des Zusatzes (13) eingeschraubt werden kann, um den Zusatz einzuziehen und zu befestigen, während das Endoskop eine Nut (18) aufweist, welche sich in Umfangsrichtung um den äußeren Umfang des Kopfteils (29) des Endoskopeinführteils (11) erstreckt, gekennzeichnet durch eine Vielzahl von radial nach innen ragenden Eingriffsgliedern (19a), welche vom Vorderende (19) des Verbindungsteils (17) hervorragen, wobei das Verbindungsteil eine Vielzahl von Schlitzen (20) aufweist, welche in axialer Richtung am vorderen Ende (19) des Verbindungsteils (17) angebracht sind, um dem Verbindungsteil eine elastische Verformbarkeit in Radialrichtung zu verleihen, und wobei die radial nach innen ragenden Eingriffsglieder (19a) dauernd in die Umfangsnut (18) eingreifen, um eine axiale Bewegung des Verbindungsteils (17) im Verhältnis zu dieser zu unterbinden.

2. Ein Endoskopgerät gemäß Anspruch 1, bei dem das genannte Verbindungsteil (17) an seiner Außenfläche hinter dem Bereich des Außengewindes (16) Mittel (21) zum Greifen mit den Fingern aufweist.

## FIG.1

## FIG.2

## FIG.3

5